# EUROPÄISCHE PATENTSCHRIFT

(11) **EP 2 671 527 B1**
(45) Veröffentlichungstag und Bekanntmachung des Hinweises auf die Patenterteilung: **15.07.2015**
(21) Anmeldenummer: 12004329.4
(22) Anmeldetag: 06.06.2012
(51) Int. Cl.: A61B 18/14, A61B 18/16, A61B 18/00

(54) **Elektroden Katheter**
Electrode catheter
Cathéter à électrode

(43) Veröffentlichungstag der Anmeldung: 11.12.2013
(73) Patentinhaber: Peter Osypka Stiftung, 79639 Grenzach-Wyhlen (DE)
(72) Erfinder: Osypka, Peter, 79639 Grenzach-Wyhlen (DE)

(56) Entgegenhaltungen:
- WO-A2-2007/008954
- US-A1- 2003 083 613
- US-A1- 2006 247 607
- US-A1- 2009 312 755

## Beschreibung

Die Erfindung betrifft einen Elektroden Katheter, dessen distales Schaft Ende eine Öffnung besitzt, durch den ein in Längsrichtung verschiebbarer innerer Katheter mit einem distalen Fixierungsmechanismus in das Gewebe einschraubbar ist und damit einen Pol darstellt. Durch Anlegen einer Hochfrequenz Spannung von ca 500 kHz zwischen den Polen des äußeren Katheters und des inneren Katheters und gleichzeitigem Zurückziehen des Katheters wird im Gewebe eine Linie von ablatierten Zellen erzeugt, die eine Hilfe bei der Therapie von Vorhofflimmern ist.

### Wie wurde das Problem bisher gelöst

Vorhofflimmern tritt relativ häufig auf. Insgesamt scheint Vorhofflimmern in den letzten Jahren zugenommen zu haben, was mit einem immer älter werdenden Patientenkollektiv erklärt wird. Vorhofflimmern führt zu einer schnellen Überleitung der Erregung auf die Ventrikel, so dass es zu akuter hämodynamischer Instabilität kommen kann. Die Beseitigung von Vorhofflimmern durch Hochfrequenz Ablation erfolgt bisher durch einen im linken Vorhof eingeführten Katheter, der nach der sogenannten "Mace Prozedur" neben den Ausgängen der Pulmonal Venen auch andere Teile im linken Vorhof ablatiert. Wichtig dabei ist es, dass die Ablation nicht punktförmig, sondern in Linien erfolgt. Die dafür notwendigen Katheter bestanden aus einer großen Anzahl von Polen und waren dementsprechend aufwendig herzustellen. Darüber hinaus ist die genaue Manipulation der Katheter sehr schwierig und erfordert daher viel Zeit. Weiterhin ist die Belastung durch Röntgenstrahlen für Patient und Arzt sehr hoch. Ein Katheter nach dem einleitenden Teil von Anspruch 1 ist aus US 2006247607 bekannt.

### Lösungsweg

Es besteht daher die Aufgabe einen Katheter der eingangs genannter Art zu schaffen, der es ermöglicht, einfacher, schneller und ohne oder mit geringer Röntgenbelastung das Vorhofflimmern beim Menschen zu beseitigen. Zur Lösung dieser Aufgabe ist es entsprechend der Definition der Erfindung in Anspruch 1 vorgesehen, dass die notwendige Ablation mit einer Katheter Anordnung, bestehend aus einem äußeren Katheter und einem inneren Katheter, die ineinander verschiebbar sind, durchgeführt wird. Der innere Katheter, der einen indifferenten Pol der Katheter Anordnung darstellt, ist mit einem Fixierungsmechanismus ausgestattet, dessen distale Spitze im Gewebe fixiert wird. Der verschiebbare äußere Katheter ist ebenfalls mit einem Elektroden Pol ausgestattet, der an der Wand des linken Vorhofs des Herzens platziert wird. Zwischen den Polen des inneren und äußeren Katheters wird eine Hochfrequenz Spannung von ca. 500 kHz angelegt, dass das darunter liegende Gewebe erwärmt. Durch Zurückziehen des äußeren Katheters während des Hochfrequenz Stromes und bei gleichzeitiger Überwachung der Temperatur durch eingebaute Temperatur Sensoren in den Polen der Katheter, wird durch die lokale Erwärmung (Ablation) des darunter liegenden Gewebes eine Linienstruktur erzeugt. Durch geschickte Manipulation der Katheter Anordnung kann im gesamten linken Vorhof die gewünschten "Ablations Linie" erzeugt werden. Diese Methode ist nicht nur auf den linken Vorhof beschränkt, sondern kann an jeder Stelle des menschlichen Körpers, wie z.B. Ventrikel, Nieren, Magen, angewendet werden.

### Vorteile

Der große Vorteil der Erfindung liegt darin, dass mit Hilfe einer einzigen zweipoligen Katheter Anordnung die gewünschte durchgehende "Linie" im linken Vorhof erzeugt werden kann.

### Zeichnungen

Weitere Einzelheiten, Merkmale und Vorteile der Erfindung lassen sich dem folgenden Beschreibungsteil entnehmen, in dem anhand der Zeichnungen und Ausführungsbeispiele die Erfindung näher erläutert wird. Sie zeigen in schematischer Darstellung in
Fig.1 Eine beispielhafte Katheter Anordnung im linken Vorhof des Herzen, die mit Hilfe einer Schraubwendel fixiert ist.
Fig.2 Eine detaillierte Darstellung von beispielhaften distalen Katheter Anordnungen mit dem dazu gehörigen inneren Katheter mit Schraubwendel oder Magnet als Fixierungs Hilfe.
Fig.3 Eine beispielhafte distale Teil Katheter Anordnung mit einer distalen Leuchtdiode (LED), Temperaturfühler und der Schraubwendel.
Fig.4 Positionierung der Katheter Anordnung an der äußeren Wand des linken Vorhofs.
Fig.5 Positionierung der Katheter Anordnung an der äußeren Wand des linken Vorhofs beim Rückzug während der Hochfrequenz Ablation.

### Beschreibung der Abbildung

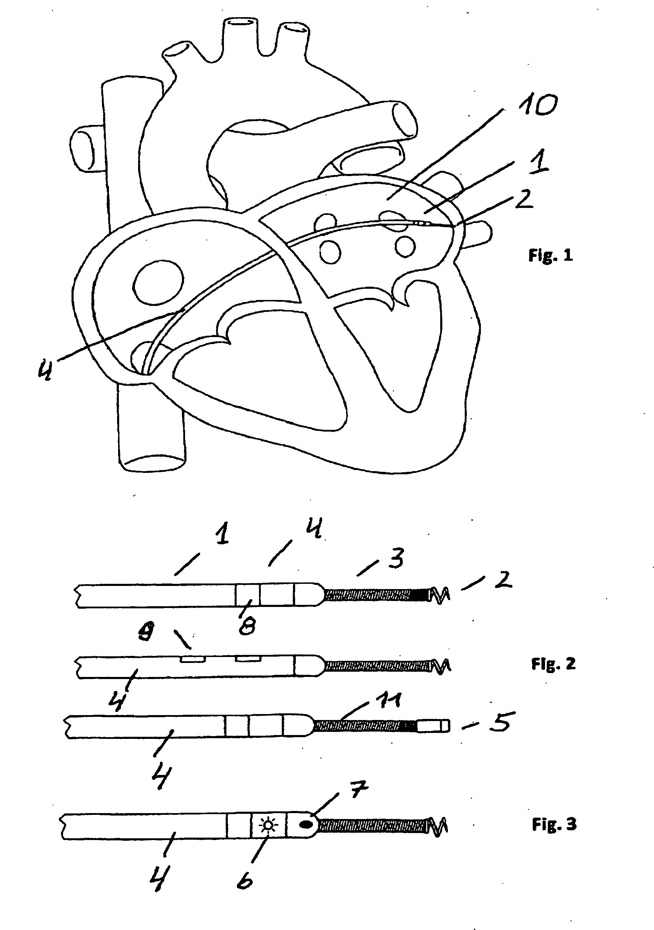

Fig.1 Zeigt eine beispielhafte Katheter Anordnung (1) im linken Vorhof (10) des Herzen, die mit Hilfe einer Schraubwendel (2) fixiert ist. Der Zugang in den linken Vorhof erfolgt routinemäßig transseptal. Zunächst wird mit Hilfe eines steuerbaren Katheters(hier nicht gezeichnet), der innere Katheter(3) bis an das Ziel im linken Vorhof eingeführt. Danach erfolgt die Fixierung des inneren Katheters mittels Fixierung der Schraube(2) an dem gewünschten Ort im linken Vorhof (10) und Entfernen des steuerbaren Katheters. Über den inneren Katheter(3) wird danach der äußere Katheter(4) eingeführt.

Fig.2 Zeigt eine detaillierte Darstellung von beispielhaften distalen Katheter Anordnungen (1) mit den dazu gehörigen inneren Kathetern(3), mit dem äußeren Katheter(4) und der Schraubwendel (2) oder Magneten (5) als Fixierungs Hilfe.
Der Pol (8) des äußeren Katheters ist aus Metall oder einem leitfähigen Kunststoff wie z.B. Polyurethan oder Silikon. Die Ausgestaltung des Pols (bzw. der Pole) ist variabel. Der Pol kann zylindrisch oder als Segment (9) ausgebildet sein. Der innere Katheter (3) enthält vorzugsweise eine torsionsstabile Litze oder Wendel(11), die elektrisch leitend ist.

Fig.3 Zeigt eine beispielhafte Katheter Anordnung (1) mit einer distalen roten Leuchtdiode (LED) (6), einem Temperatur Fühler (7) und der Schraubwendel (2). Der Temperatur Fühler dient zur Kontrolle der Gewebe Temperatur während der Ablation. Die rote LED zeigt ohne Röntgen Belastung des Patienten über einen Sensor z.B. im Ösophagus oder in einer Vene, visuell die Position des Katheters an.

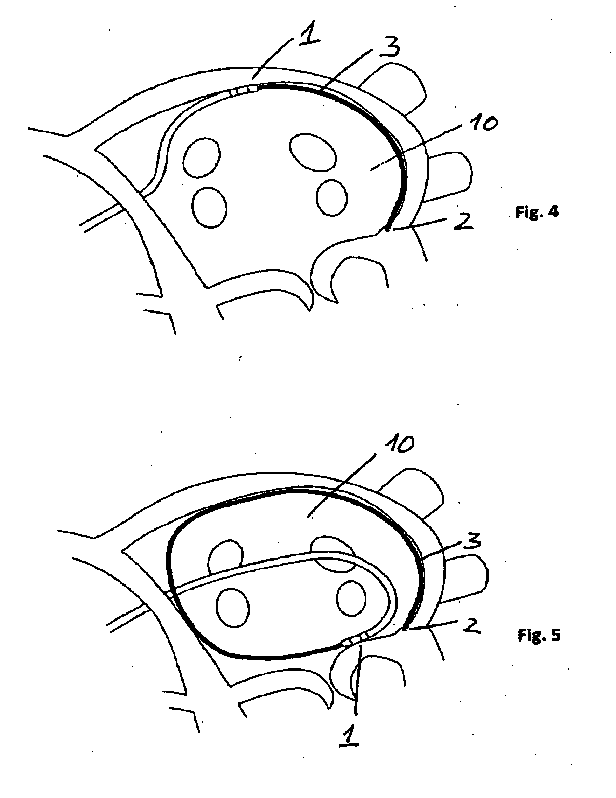

Fig.4 Zeigt die Position der Katheter Anordnung (1) an der äußeren Wand des linken Vorhofs (10) beim Rückzug während der Hochfrequenz Ablation.

Fig.5 Zeigt eine weitere Position der Katheter Anordnung (1) an der äußeren Wand des linken Vorhofs(10) beim Rückzug während der Hochfrequenz Ablation.
Durch Drehen um 180° des äußeren Katheters(4) erfolgt ein Positionswechsel in der dargestellten Form. Je nachdem an welcher Stelle die distale Fixierung erfolgt und in welcher Position das 180° Drehen stattfindet, kann jeder Bereich im linken Vorhof (10) erreicht und als Linie ablatiert werden. Damit ist es auch möglich, die Ablation um die Pulmonal Venen durchzuführen. Um eine besondere Tiefenwirkung der Ablations Linie zu erreichen, kann man mit Einführen von Kochsalzlösung zwischen Fixierungs- und Ablations Katheter eine Kühlung der Pole erreichen.

## Patentansprüche

1. Elektroden-Katheter, bestehend aus einem inneren (3) und einem äusseren Elektroden-Katheter (4), der mindestens einen Pol (8) zur Aufnahme und Abgabe elektrischer Energie besitzt, wobei der äußere Katheter (4) ein Lumen aufweist in dem sich der innere Katheter (3) erstreckt und wobei der innere und der äußere Katheter gegeneinander verschiebbar sind, wobei der innere Katheter (3) elektrisch leitend ist und einen indifferenten Pol darstellt, und zwischen dem inneren Katheter (3) und dem Pol (8) ein HF-Strom fliessen kann **dadurch gekennzeichnet, dass** der innere Kather (3) distal eine Schraubwendel oder einen Magneten als Fixierungselement enthält

2. Elektroden Katheter nach Anspruch 1und 2
**dadurch gekennzeichnet,**
**dass** der äußere Katheter(4) distal eine Öffnung für die Aufnahme des inneren Katheters(3) enthält.

3. Elektroden Katheter nach Anspruch 1 bis 3
**dadurch gekennzeichnet,**
**dass** der äußere Katheter(4) distal mindestens einen Temperaturfühler(7) und /oder eine rote Leuchtdiode (LED)(6) enthält.

4. Elektroden Katheter nach Anspruch 1 bis 4
**dadurch gekennzeichnet,**
**dass** der äußere Katheter(4) distal mindestens einen Pol(8) zur Aufnahme und Abgabe elektrischer Energie besitzt.

5. Elektroden Katheter nach Anspruch 1 bis 5
**dadurch gekennzeichnet,**
**dass** die Pole des äußeren Katheters(4) aus Metall oder leitfähigem Kunststoff hergestellt sind.

6. Elektroden Katheter nach Anspruch 1 bis 6
**dadurch gekennzeichnet,**
**dass** die Pole des Katheters(8) zylindrisch oder als Segmente(9) ausgebildet sind.

7. Elektroden Katheter nach Anspruch 1 bis 7
**dadurch gekennzeichnet,**
**dass** der Aufbau des inneren Katheters mindestens eine Litze oder Wendel(11) enthält.

8. Elektroden Katheter nach Anspruch 1 bis 8
**dadurch gekennzeichnet,**
**dass** der äußere Katheter(4) aus einem flexiblen Kunststoff hergestellt ist.

## Claims

1. An electrode catheter device comprising:
an inner electrode catheter (3); and an outer electrode catheter (4), said outer electrode catheter (4) comprising at least one pole (8) for receiving and transmitting electrical power whereby the outer electrode catheter (4) comprises a lumen which can receive the inner electrode catheter (3) therein, and whereby the outer electrode catheter is adjustable or movable relative to the inner electrode catheter,
wherein the inner electrode catheter (3) is electrically conductive thus representing an indifferent pole to let HF current flow between the inner catheter (3) and the pole (8), the inner electrode catheter (3) distally comprises a fixation element, said fixation element being a screwing coil or a magnet.

2. The electrode catheter device according to claim 1, whereby the outer electrode catheter (4) distally comprises an opening to receive the inner electrode catheter (3).

3. The electrode catheter device according to claim 1 or 2, wherein the outer electrode catheter (4) distally comprises at least one temperature sensor (7) and /or a red light emitting diode (LED) (6).

4. The electrode catheter device according to any one of claims 1 to 3, whereby the outer electrode catheter (4) distally comprises at least one pole (8) for receiving and transmitting electrical power.

5. The electrode catheter device according to any one of claims 1 to 4, wherein the electrode pole of the outer electrode catheter (4) is made of metal or of conductive plastic.

6. The electrode catheter device according to any one of claims 1 to 5, wherein the electrode pole (8) of the outer electrode catheter is designed in cylindrical shape or in shape of a segment (9).

7. The electrode catheter device according to any one of claims 1 to 6, wherein the inner electrode catheter is a strand or a coil (11).

8. The electrode catheter device according to any one of claims 1 to 7, wherein the outer electrode catheter (4) is made of flexible plastic.

## Revendications

1. Cathéter à électrodes comprenant un cathéter à électrodes intérieur (3) et un cathéter à électrodes extérieur (4) dans lequel le cathéter à électrodes extérieur (4) comporte au moins un pôle (8) pour recevoir et délivrer de l'énergie électrique et une lumière qui peut recevoir le cathéter à électrodes intérieur (3) dans celui-ci, dans lequel le cathéter à électrodes extérieur et le cathéter à électrodes intérieur sont déplaçable l'une par rapport à l'autre, dans lequel le cathéter à électrodes intérieur est électriquement conducteur et forme une électrode indifférente, dans lequel entre le cathéter intérieur (3) et le pôle (8) du cathéter extérieur (4) peut circuler le courant HF; dans lequel le cathéter comprend un fixation disposé distalement, ledit fixation étant une hélice ou un aimant.

2. Cathéter à électrodes selon la revendication 1 **caractérisé en ce que** le cathéter extérieur (4) comprend une ouverture situées distalement pour recevoir le cathéter intérieur (3).

3. Cathéter à électrodes selon l'une des revendications 1 et 2 **caractérisé en ce que** le cathéter extérieur (4) est équipé distalement au moins d'une sonde de température (7) et / ou d'une diode électroluminescente rouge (LED) (6).

4. Cathéter à électrodes selon l'une des revendications 1 à 3, **caractérisé en ce que** le cathéter à électrodes extérieur (4) comporte au moins un pôle (8) pour recevoir et délivrer de l'énergie électrique.

5. Cathéter à électrodes selon l'une des revendications 1 à 4, **caractérisé en ce que** les pôles du cathéter extérieur (4) sont réalisés en métal ou d'une matière plastique conductrice de l'électricité.

6. Cathéter à électrodes selon l'une des revendications 1 à 5, **caractérisé en ce que** les pôles du cathéter (8) sont réalisés avec une forme cylindrique ou en forme d'un segment (9).

7. Cathéter à électrodes selon l'une des revendications 1 à 6, **caractérisé en ce que** le cathéter intérieur comprend au moins un toron ou un filament helicoidale (11).

8. Cathéter à électrodes selon l'une des revendications 1 à 7, **caractérisé en ce que** le cathéter extérieur (4) est réalisé en une matière plastique flexible.
